# EUROPÄISCHE PATENTANMELDUNG

(11) **EP 0 062 319 A1**
(43) Veröffentlichungstag der Anmeldung: **13.10.1982**
(21) Anmeldenummer: 82102800.8
(22) Anmeldetag: 02.04.1982
(51) Int. Cl.: A61K 31/415

(54) **3-Methyl-1-(2-(2-naphthyloxy)-ethyl)-2-pyrazolin-5-on zur Behandlung der Metastase und des Wachstums von Tumor-Zellen**

(30) Priorität: 07.04.1981 US 251864
(71) Anmelder: BAYER AG, 51368 Leverkusen (DE)
(72) Erfinder: Busse, Wolf-Dieter, Dr., D-5600 Wuppertal 1 (DE); Honn, Kenneth V., Dr., Michigan 48236 (US); Möller, Eike, Dr., D-5600 Wuppertal 1 (DE); Seuter, Friedel, Dr., D-5600 Wuppertal 1 (DE)

(57) **Zusammenfassung**

Eine therapeutische Methode zur Reduzierung der Metastase und des neoplastischen Wachstums wird beschrieben. Die Methode umfasst die Verabreichung einer therapeutisch wirksamen Menge 3-Methyl-1-[2-(2-naph- thyloxy)-ethyl]-2-pyrazolin-5-on oder eines pharmazeutisch unbedenklichen Salzes.

## Beschreibung

Das primäre Ziel einer Krebsbehandlung ist die Behandlung und vollständige Ausrottung des Wachstums des primären Tumors. Gleichzeitig mit dieser Behandlung ist die Verhinderung der Metastasierung notwendig, die als die Absiedelung primärer Tumorzellen und deren nachfolgendes Eindringen in das Lymphsystem oder die Blutgefäße zur Verschleppung definiert werden kann. Eine solche Ausbreitung kann durch Anhaften an den endothelialen Wandungen und anschließendes Durchdringen derselben, Ansiedelung sekundärer Tumoren in den perivaskulären Geweben und schließlich Verbreitung der Tumorzellen zu entfernteren Stellen hin erfolgen. Obwohl über die klinischen Manifestationen des metastatischen Prozesses viel bekannt ist, weiß man nur wenig über die an der Metastase beteiligten biochemischen, immunologischen, genetischen und hormonalen Mechanismen. In diesem Sinne kann die Metastase als eine einzelne Erscheinung betrachtet werden, die auf einer Folge komplizierter Vorgänge beruht.

Wegen der Bedeutung sowohl der Behandlung des Wachstums des primären Tumors als auch der Verhütung der Metastase haben die Krebsforscher umfangreiche Untersuchungen durchgeführt, um die mit Tumorwachstum und Metastase verbundenen Wechselwirkungen aufzuklären.

Eine der biologischen Eigenschaften, die Tumorzellen zu besitzen scheinen, ist die Fähigkeit, mit Wirts-Blutplättchen in Wechselwirkung zu treten und sich an diese anzuheften und dadurch das Potential des Tumors zu verstärken, sich im Mikrogefäßsystem festzusetzen und an dem vaskulären Endothel zu haften. Als eine weitere Möglichkeit wurde vorgeschlagen, daß die Tumorzellen nach ihrem Festsetzen die Bildung sie umgebender, als Schutz wirkender Plättchen-Thromben auslösen können. Damit eine erfolgreiche Metastase stattfinden kann, müssen die metastatischen Zellen zunächst an dem vaskulären Endothel sich festsetzen und dort haften und intravaskulär bleiben, bis es in das umgebende Gewebe eindringt.

Wegen der Ähnlichkeiten zwischen dem an dem Festsetzen und Anhaften der Tumorzellen an dem Endothel beteiligten Prozeß und der Bildung von Nicht-Tumor-Thromben haben viele Forscher geschlossen, daß Plättchen auf irgendeine Weise dabei eine Rolle spielen. Wegen dieser Beteiligung der Plättchen wurden zahlreiche Untersuchungen durchgeführt, um die Wirkung einer Therapie mittels Antikoagulantien auf die Metastase zu bestimmen. Die nachstehend zitierten Untersuchungen umfaßten die Verabreichung antikoagulierender Verbindungen, die potente Inhibitoren für eine Plättchen-Aggregierung sind. Die Ergebnisse sind bis heute widersprüchlich.

Von Heparin wurde berichtet, daß es die Metastase sowohl vermindert als auch erhöht, insbesondere in der Lunge [vgl. Cell Biol.Intl.Rep. 2, 81-86 (1963), und Arch. Surg. 91, 625-629 (19658. Aspirin lieferte gemischte Ergebnisse [vgl. Eur.J.Cancer 8, 347-352 (1972), und Intl.J.Cancer 11, 704-718 (1973)]. Für Warfarin wurde gezeigt, daß es signifikante antimetastatische Wirkungen nach intravenöser Injektion von Tumorzellen und in spontan metastasierenden Tumoren hervorruft [vgl. Cancer 35, 5-14 (1975), und Cancer Res. 37, 272-277 (1971)]. Es wurde gezeigt, daß eine durch intravenöse Verabreichung von B-16ₐ-Melanom-Zellen induzierte Metastase durch Ver- abreichung des Antikoaguliermittels Prostacyclin verhindert werden kann [vgl. Cell Biol. 87, 649](1980).

Eine übersicht über die Verwendung von Antikoagulantien zur Tumor-Therapie findet sich bei M. B. Donati et al, Malignancy and the Hemostatic System, Seiten 103-120, Raven Press, 1981.

Es wurde die Vermutung geäußert, daß der Einsatz der Therapie mittels Antikoagulantien zum Teil deshalb weniger als zufriedenstellend verlaufen ist, weil es den verwendeten Antikoaguliermitteln an Spezifität fehlt, und weil einige der Mittel auf den Tumorzellen selbst Wirkungen hervorrufen, die insgesamt die gewünschte Wirkung auf die Blutplättchen und damit auf die Metastase aufheben können.

Gemäß der vorliegenden Erfindung hat sich die in der US-PS 4 053 621 offenbarte und als therapeutisch wirksames antithrombotisches Mittel beanspruchte Verbindung 3-Methyl-1-[2-(2-naphthyloxy)-ethyl]-2-pyrazolin-5-on als ein potentes antimetastatisches Mittel mit begleitender antineoplastischer Wirksamkeit erwiesen.

Die vorliegende Erfindung ist auf eine therapeutische Methode zur Verminderung der Metastase und des neoplastischen Wachstums in Säugern gerichtet. Diese Methode umfaßt die Verabreichung einer therapeutisch wirksamen Menge 3-Methyl-1-[2-(2-naphthyloxy)-ethyl]-2-pyrazolin-5-on an den Säuger.

Wie in der US-PS 4 053 621 beschrieben wurde, kann die in der vorliegendenErfindung verwendete Methylpyrazolon-Verbindung (dargestellt durch die folgende Formel I) auf verschiedenen Synthesewegen hergestellt werden, wie nachstehend erläutert wird. Nach dem Verfahren A wird 2-(2-Naphthyloxy)-ethylhydrazin mit einem Acetessigsäure-Derivat umgesetzt; nach dem Verfahren B wird 3-Methylpyrazolinon-(5) mit einem 2-(2-Naphthyloxy)-ethyl-Derivat umgesetzt; nach dem Verfahren C wird 2-(2-Naphthyl- oxy)-ethylhydrazin mit einem Tetrolsäure-Derivat umgesetzt.

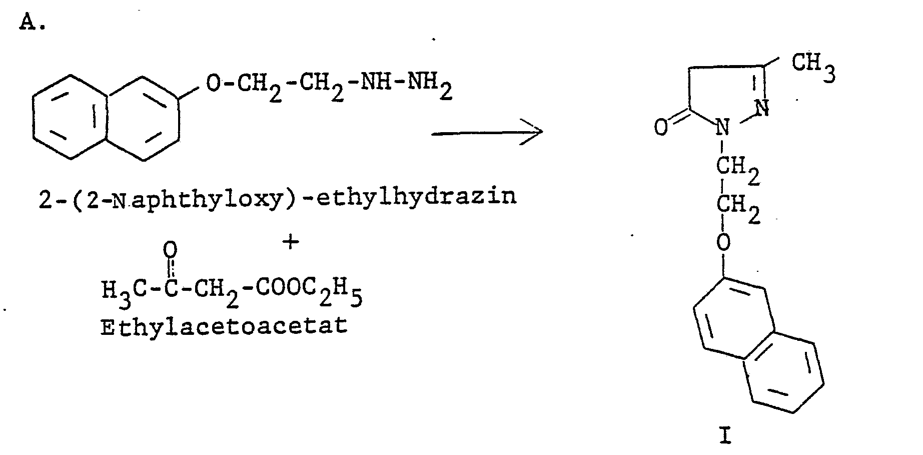

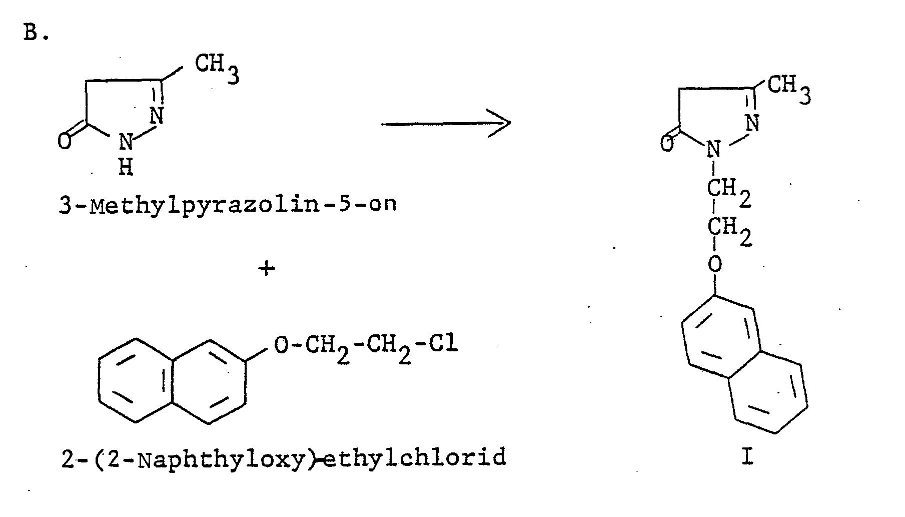

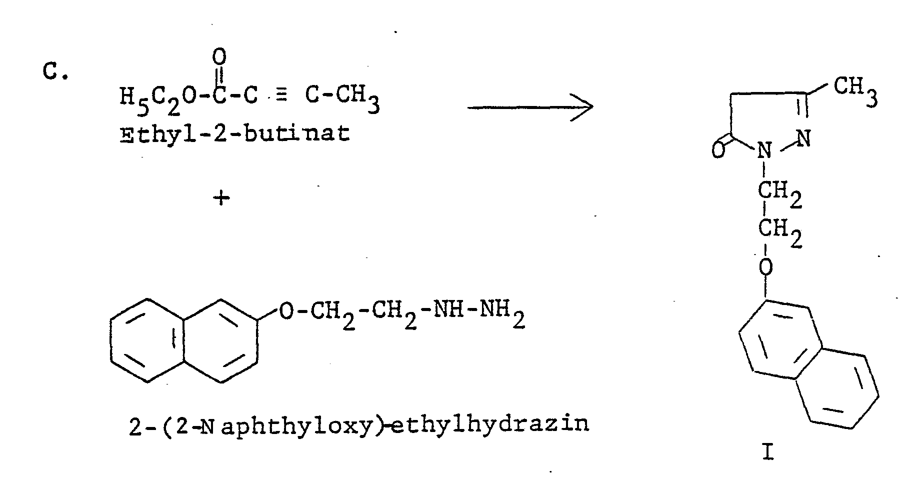

Zu den verwendbaren Verdünnungsmitteln zählen sämtliche inerten organischen Lösungsmittel, wahlweise verdünnt mit Wasser, z.B. Kohlenwasserstoffe wie Benzol und Toluol, Halogenkohlenwasserstoffe wie Methylenchlorid, Alkohole wie Methanol und Ethanol, und organische Basen wie Pyridin und Picolin.

Basische oder saure Kondensationsmittel können verwendet werden, und die Reaktionstemperatur kann zwischen 10°C und 200°C variiert werden. Die Verbindung kann in einfacher Weise mittels üblicher Methoden durch Umkristallisieren aus einem geeigneten Lösungsmittel gereinigt werden.

In der vorliegenden Beschreibung bezeichnet der Ausdruck "pharmazeutisch unbedenkliches Verdünnungsmittel oder Trägermaterial" eine nicht-toxische Substanz, die nach dem Vermischen mit dem Wirkstoff oder mit Wirkstoffen diesen oder diese in einer für die Verabreichung geeigneten Form liefert. Die Bezeichnung schließt vorzugsweise Wasser und üblicherweise bei der chemischen Synthese verwendete organische Lösungsmittel mit niederem Molekulargewicht aus, außer bei Vorliegen anderer pharmazeutisch erforderlicher Bestandteile wie Salze in den richtigen Mengen, um eine isotonische Zubereitung herzustellen, Puffer, oberflächenaktive Mittel, Farb- und Geschmacksstoffe und Konservierungsmittel. Beispiele für geeignete feste und flüssige Verdünnungsmittel und Trägermaterialien sind die folgenden: Wasserhaltige Puffermittel, die durch Zusatz von Glucose oder Salzen isotonisch gemacht werden können; nicht-toxische organische Lösungsmittel wie Paraffine, pflanzliche öle, Alkohole, Glycole; gemahlene Natursteinmaterialien (beispielsweise Kaoline, Aluminiumoxide, Talkum oder Kreide); synthetische Gesteinpulver (beispielsweise hochdisperse Kieselsäure oder Silicate); und Zucker.

Die orale Verabreichung kann unter Verwendung fester und flüssiger Dosierungsformen wie Pulver, Tabletten, Dragees, Kapseln, Granulat, Suspensionen, Lösungen und dergleichen vorgenommen werden. Wo angezeigt können die Dosierungseinheiten für die orale Verabreichung mikroverkapselt werden, um die Abgabe hinauszuzögern oder über längere Zeit hinweg zu verlangsamen, wie beispielsweise durch Überziehen oder Einbetten von teilchenförmigem Material in Polymere, Wachs oder dergleichen.

Die parenterale Verabreichung kann unter Verwendung flüssiger Dosierungsformen wie steriler Lösungen und Suspensionen erfolgen, die für die subkutane, intramuskuläre oder intravenöse Injektion bestimmt sind. Diese werden durch Suspendieren oder Auflösen einer abgemessenen Menge der Verbindung in einem zur Injektion geeigneten nicht-toxischen flüssigen Streckmittel wie einem wäßrigen oder öligen Medium und Sterilisierung der Suspension oder Lösung hergestellt. Stabilisatoren, Konservierungsmittel und Emulgatoren können ebenfalls zugesetzt werden.

Im allgemeinen beträgt die auf das Körpergewicht bezogene Tagesdosis für die parenterale Verabreichung 0,01 bis 50 mg/kg, vorzugsweise 0,1 bis 10 mg/kg, und für die orale Verabreichung 0,1 bis 500 mg/kg, vorzugsweise 0,5 bis 100 mg/kg.

Zur Bestimmung der antimetastatischen und antineoplastischen Eigenschaften von 3-Methyl-1-[2-(2-naphthyloxy)-ethyl]-2-pyrazolin-5-on wurde die folgende Arbeitsweise angewandt. Das Test-Schema bedient sich zweier nicht miteinander in Beziehung stehender Typen von Mäuse-Tumoren (eines Melanoms und eines Karzinoms), um die Möglichkeit weitestgehend auszuschalten, daß die erhaltenen Ergebnisse "einzigartig" in bezug auf einen einzigen Tumortyp sind. Diese beiden Tumoren werden routinemäßig für grundlegende Untersuchungen über den Mechanismus der Metastase und der antineoplastischen Wirkung.verwendet.

### A. In vivo-Erhaltung der Tumor-Linien

Subkutanes, amelanotisches B-16 -Melanom (B-16ₐ) und Lewis-Lungenkarzinom (3LL) wurden von der Division of Cancer Treatment, (NCI), Animal and Human Tumor Bank, Mason Research Institute, Worcester, Massachusetts, USA, erhalten. Beide Tumor-Typen wurden nach Erhalt vier Passagen unterzogen. Eine Passage umfaßte die Implantation eines Tumorwürfels von 2 x 2 mm in die rechte Achselhöhlenregion (unter Benutzung einer 0,33 mm- = 13 gauge -Trokarnadel) männlicher, syngeneischer Wirtsmäuse L(C57BL/6J; Jackson Laboratory -StammJ.

Die Wirtsmäuse wogen zwischen 17 und 22 g (sie waren etwa 28 Tage alt) und wurden hinsichtlich der Lichteinwirkungsdauer, Diät, Temperatur etc. unter identischen Bedingungen gehalten.

Die transplantierten Tumoren wurden im Anschluß an die Implantation in den syngeneischen Wirtsmäusen etwa 14 Tage lang wachsen gelassen.

### B. Isolierung und Suspension der Tumorzellen

Tumorzellen wurden dann durch aseptische Entnahme von von den Wirtsmäusen erhalten und unter Anwendung sequenzieller Collagenase-Verdauung dispergiert, wie unten beschrieben. Die entnommenen Tumoren wurden in Würfel (4 x 4 mm) zerteilt, und das gewürfelte Gewebe wurde auf 6 bis 8 sterile Polycarbonat-Erlenmeyerkolben aufgeteilt (etwa 500 mg/Kolben). Ein Anteil von 10 ml einer "Tumordispersionslösung" (TDS) wurde in jeden Kolben hinzugefügt.

Die TDS wurde durch Zusammenmischen der Zusammensetzung A und der Zusammensetzung B hergestellt, die nachstehend beschrieben werden.
Zusammensetzung A (bezogen auf 1 1) 9,5 g/1 Steriles Eagle's "Minimum Essential Medium" (MEM) (im Handel erhältlich bei Gibco, Grand Island, New York), 10 ml/l Nichtessentielle Aminosäuren (Gibco), 10 ml/l Natriumpyruvat, 0,35 g/l Natriumhydrogencarbonat (15 mM), 5,9 g/l HEPES (25 mM) = 4-(2-Hydroxyäthyl)-1-piperazinethan-sulfonsäure (ein organischer Puffer; im Handel erhältlich bei Sigma Chemical, St. Louis, Missouri), 150 Einh./ml Penicillin, 100 µg/ml Neomycin-sulfat.

Die Antibiotika wurden zugesetzt, um sicherzustellen, daß keine bakterielle Verunreinigung stattfand.

Zusammensetzung B ist eine trockene Mischung enthaltend Collagenase mit wenig Clostripain und anderer proteolytischer Aktivität; Desoxyribonuclease (DNase), um das von den beschädigten Zellkernen freigesetzte Desoxyribonukleoprotein aufzulösen; Lima-Bohnen- oder Sojabohnen-Trypsininhibitoren, um jegliche tryptische Restwirkung auszuschließen; menschliches Serumalbumin, um nicht-spezifische Protease-Aktivität auszuschalten und Peroxysowie Hydroperoxy-Fettsäuren zu absorbieren, die von beschädigten Membranen freigesetzt wurden.

### Zusammensetzung B

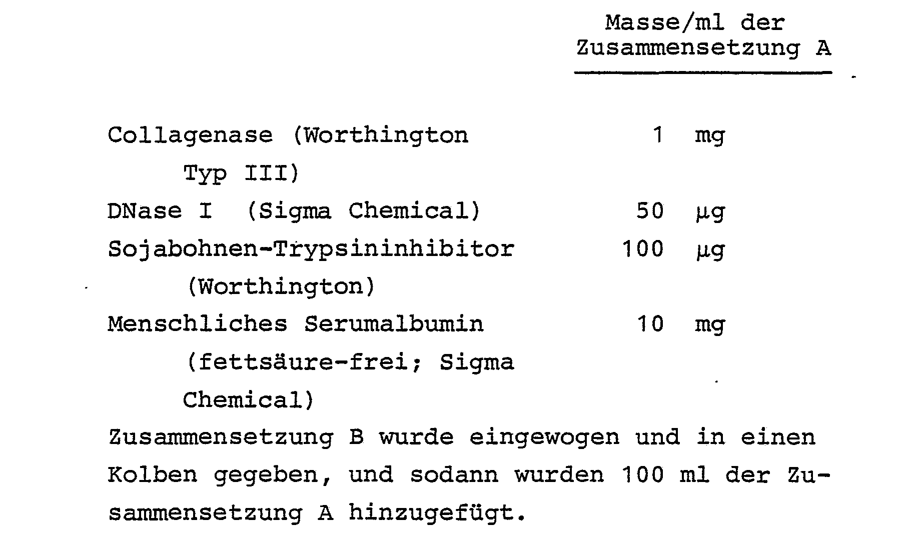

Das gewürfelte Gewebe in der TDS wurde dann unter Luft in einem Dubnoff-Stoffwechsel-Schüttler (90 Schwingungen /min) dispergiert (30 min; 37°C). Die Überstände wurden durch ein Seihtuch in sterile 50 ml-Rundboden-Zentrifugenröhrchen aus Polycarbonat gesammelt und 10 min (25°C) mit 900/min (100 g) in einem Sorvall SS-34 -Rotor zentrifugiert. Nach dem Zentrifugieren wurde die überstehende Fraktion verworfen. Das erhaltene zelluläre Material (Pellets) wurde zweimal mit MEM-Lösung gewaschen, wieder in MEM suspendiert und bei 4°C gehalten.

Zu dem verbleibenden gewürfelten Gewebe wurde ein Anteil von 10 ml TDS hinzugefügt, und das Gewebe wurde in einem Stoffwechselschüttler inkubiert, wie oben beschrieben, außer daß die Zeitdauer 60 min betrug. Das Zentrifugieren wurde wiederholt, und die wieder suspendierten Zellen wurden vereinigt.

Die Lebensfähigkeit der Zellen wurde nach der Vitalfarbstoff-Ausschlußmethode (vital dye exclusion method)[vgl. Exptl. Cell Res. 13, 341-347 (1957)1 bestimmt. Die Zellenzahl (cell count) wurde in einem Hämocytometer bestimmt. Die stromalen Zellverunreinigungen, z.B. Makrophagen, rote Blutkörperchen, etc. wurden durch visuelle Prüfung unter dem Mikroskop bestimmt. Die schließlich erhaltene Zellsuspension bestand zu mehr als 99 % aus monodispersen Zellen mit annähernd 25 % Verunreinigung durch stromale Wirtszellen. Typische Ausbeuten von 3,0 g eines B-16 - oder 3LL-Tumors lagen zwischen 9 · 10⁸ und 1,3 ' 10⁹ lebensfähiger Tumorzellen.

Die Zellsuspensionen der Endstufe wurden dann zur endgültigen Abtrennung der Tumorzellen einer zentrifugalen Elutriation unterworfen. Bei der zentrifugalen Elutriation werden die Zellen im Inneren einer Trennkammer der Einwirkung zweier entgegengesetzter Kräfte unterworfen, nämlich einem durch einen umlaufenden Rotor erzeugten Zentrifugalfeld und einem Flüssigkeitsgegenstrom in der entgegengesetzten (zentripetalen) Richtung. Eine in einem Medium suspendierte Probe tritt in die Trennkammer ein. Jede Zelle neigt dazu, zu einer Zone zu wandern, in der ihre Sedimentationsgeschwindigkeit genau durch die Durchflußgeschwindigkeit der Flüssigkeit durch die Trennkammer hindurch ausgeglichen wird. Die Geometrie der Kammer erzeugt einen Gradienten der Strömungsgeschwindigkeiten vom einen Ende zum anderen; Zellen mit einem weiten Bereich unterschiedlicher Sedimentationsgeschwindigkeiten können in Suspension gehalten werden. Durch Erhöhung der Strömungsgeschwindigkeit der Elutriationsflüssigkeit (Trennmedium) in Stufen oder durch Erniedrigung der Rotor-Geschwindigkeit können aufeinanderfolgende Populationen mit relativ homogenen Zellgrößen aus der Kammer herausgewaschen werden. Jede Population enthält Zellen, die größer oder dichter (d.h. rascher sedimentierend) sind als diejenigen der vorhergehenden Fraktion.

Die zentrifugale Elutriation wurde durch Suspension der Tumorzellen in einer"Tumor-Resuspensionslösung" (TRS) durchgeführt, die die folgende Zusammensetzung, bezogen auf 1 1, hatte.
9,5 g/1 Steriles Eagle's "Minimum Essential Medium" (MEM) (Gibco),
10 ml/l Nichtessentielle Aminosäuren (Gibco),
10 ml/l Natriumpyruvat,
0,35 ml/1 Natriumhydrogencarbonat (15 mM),
5,9 g/l HEPES (25 mM) (Sigma Chemical),
150 Einh./ml Penicillin,
100 µg/ml Neomycin-sulfat.

Die Suspension wurde unter Verwendung eines Beckman-JE-6 -Elutriator-Rotors elutriiert, der in einer Beckman J-2-21 -Zentrifuge mit 2000/min bei 25°C betrieben wurde.

Ein Trennmedium aus "Hank's Balanced Salt Solution" wurde unter Benutzung einer Cole Palmer Master Flex-Pumpe mit einem Pumpenkopf Nr. 7014 durch das System gepumpt. Der Pumpensteuerkasten wurde mittels eines Potentiometers mit 10 Umdrehungen modifiziert [vgl. Anal. Biochem. 98, 112-115 (1979)]. Die Durchflußgeschwindigkeit wurde mit Hilfe eines Brooks-Doppelkugelströmungsmessers gemessen.

Hank's abgestimmte Salzlösung wurde durch Ansetzen von 900 ml Lösung der nachstehenden Zusammensetzung und Vermischen mit CaCl₂^{·}2 H₂0 wie anschließend beschrieben hergestellt.
80 g NaCl
4 g KC1
0,98 g MgSO₄
0,48 g Na₂H^{P0}₄
0,60 g KH₂^{P0}₄
1,85 g CaCl₂^{·}2 H₂0 wurden zu 100 ml Lösung aufgefüllt, und diese wurde mit den vorbezeichneten 900 ml vermischt.

Etwa 1 x 10⁹ Zellen wurden durch ein Y-Paßstück im Zuleitungssystem in die Mischkammer injiziert. Nach einer Zeit von 15 min zur Gleichgewichtseinstellung wurden die Zelltrümmer mit einer Strömungsgeschwindigkeit von 9 x 10 ml/min eluiert. Die Tumorzellen wurden in 6 Fraktionen von jeweils 50 ml mit Strömungsgeschwindigkeiten von etwa 12 bis 18 ml/min eluiert. Die die Tumorzellen enthaltenden Fraktionen 2 bis 5 wurden vereinigt, erneut zentrifugiert (100 g) und in 1 bis 2 ml der oben beschriebenen TRS wieder suspendiert. Im allgemeinen wurden zwischen 70 und 75 % der in die Mischkammer injizierten Zellen zurückgewonnen.

### C. Wirkung von 3-Methyl-1-[2-(2-naphthyloxy)-ethyl]-2-pyrazolin-5-on auf Wachstum und Metastase von Tumorzellen

Die auf diese Weise erhaltenen B-16ₐ-Melanom- und Lewis- Lungenkarzinom-Zellen wurden wie nachstehend beschrieben zur Prüfung der antimetastatischen und antineoplastischen Wirksamkeit der Methylpyrazolon-Verbindung verwendet.

### (1) Metastase

Wie bereits weiter oben festgestellt wurde, ist die Metastase eine einzelne Erscheinung, die durch eine Reihe komplizierter Vorgänge dargestellt wird. Gegenwärtig finden zwei "Modell"-Systeme für die Untersuchung der in vivo-Metastase verbreitete Anwendung. Das erste Modellsystem umfaßt die subkutane Injektion der Tumorzellen in das Tier. Die subkutane Injektion der Tumorzellen und anschließende Entwicklung eines Primärtumors, gefolgt von spontaner Metastase, wird als "volle" Metastase betrachtet. Ein anderes Modellsystem umfaßt die Injektion der Tumorzellen durch die Schwanzvene. In Anbetracht der Komplexität der Metastase gilt die Schwanzvenen-Injektion als künstliches und nur partielles Modellsystem, da es nur die Vorgänge während des letzteren Teils der Metastase wiedergibt. Das Schwanzvenen-Modellsystem gilt jedoch auch als außerordentlich günstig im Hinblick auf eine Standardisierung der Versuchsbedingungen [vgl."Methods in Cancer Research", Kapitel VII, Academic Press, Inc., 1978].

Als Kontrolltiere wurden (unbehandelte) C57B1/6J-Mäuse nach folgender Arbeitsweise auf volle Metastase untersucht. Zellsuspensionen der B-16 - und 3LL-Karzinom-Zellen, die wie oben unter A und B beschrieben erhalten worden waren, wurden subkutan in die rechte Achselhöhlenregion der männlichen C57BL/6J-Mäuse injiziert (0,66 mm- = 26 gauge -Nadel; 0,2 ml). Injiziert wurden verschiedene Mengen der Zellsuspensionen im Bereich von 1 x 10⁵ bis 1 x 10⁶ Zellen. Versuche zur partiellen Metastase wurden in der Weise durchgeführt, daß Tumorzellen über die Schwanzvene in die (unbehandelten) Kontroll-Mäuse injiziert wurden. Die Tiere wurden unter identischen Bedingungen der Temperatur, Lichteinwirkungsdauer, Ernährung etc. gehalten. Nach einem Beobachtungszeitraum von 17 bis 30 Tagen wurden die für die volle Metastase und für die partielle Metastase verwendeten Tiere getötet, und die Lungen, Leber, Nieren, Milz und Gehirn-Gewebe wurde entnommen.

Das entnommene Gewebe wurde in Bouin-Lösung fixiert.

Die Zahl der metastatischen Knötchen in jedem Organ wurde mit Hilfe eines Bausch-und-Lomb Stereo-Zoom-Mikroskops bestimmt. Die Untersuchung der Kontroll- Mäuse auf metastatische Knötchen ergab bei 100 % der Tiere positive Befunde in bezug auf metastatische Lungentumoren; die Inkubationszeit für die Erzeugung solcher Metastasen betrug für die 3LL-Tumorzellen 17 bis 21 Tage und für die B-16 a -Tumorzellen 23 bis 30 Tage. In Leber, Nieren, Milz oder Gehirn-Gewebe wurden keine sichtbaren Knötchen beobachtet.

### (2) Antineoplastische Wirksamkeit

Die antineoplastische Wirksamkeit wurde in vitro durch Messung der DNS-Synthese sowohl von B-16ₐ- als auch von Lewis-Lungenkarzinom-Zellen durch eine Methode gemessen, der die Thymidin-Aufnahme zugrundeliegt. Zellen, die in Vorbereitung der Zellteilung DNS synthetisieren, sind durch ihre Fähigkeit gekennzeichnet, Thymidin aufzunehmen. Demnach ist die Zell-Proliferation mit der DNS-Synthese verbunden. Wenn die Menge der durch die Tumorzellen synthetisierten DNS vermindert wird, so ist das ein Anzeichen dafür, daß die Zellteilung und damit das Tumorwachstum gehemmt worden ist.

Die antineoplastische Wirksamkeit wurde außerdem durch direkte Messung des Wachstums der Tumorzellen in vitro in Gewebekulturen bestimmt. Diese Methode bedient sich des "Plating" oder Aufimpfens einer bekannten Anzahl von Tumorzellen auf ein Wachstumsmedium und der Bestimmung der Wirkung der Anwesenheit der zu prüfenden Verbindung auf die Tumorzellen-Proliferation. Schließlich wurde die antineoplastische Aktivität in vivo in der Weise bestimmt, daß den Mäusen subkutan Tumorzellen injiziert wurden und das Auftreten von Tumorzellenwachstum sowie das Gewicht der wachsenden Tumorzellen in unbehandelten Kontrolltieren und in mit der Pyrazolon-Verbindung behandelten Tieren bestimmt wurden.

Die Wirkung von 3-Methyl-1-[2-(2-naphthyloxy)-ethyll-2-pyrazolin-5-on auf die durch Schwanzvenen-Injektion von Tumorzellen hervorgerufene Metastase bzw. auf die volle Metastase aufgrund der subkutanen Injektion von Tumorzellen wird in den Beispielen 1 bzw. 2 gezeigt.

### Beispiel 1

3 mg 3-Methyl-1-[2-(2-naphthyloxy)-ethyl]-2-pyrazolin-5-on wurden in 0,6 ml absolutem Ethylalkohol suspendiert. Das suspendierte Pyrazolon wurde durch Einstellen des pH auf 9,5 mit NaOH gelöst. Die endgültige Konzentration des Pyrazolons wurde durch Verdünnen der Lösung mit normaler Kochsalzlösung (0,9 % NaCl) eingestellt.

Syngeneische C57BL/6J Wirtsmäuse erhielten während eines Zeitraums von drei Tagen eine Injektion auf täglicher Basis von 0,02 und 0,08 mg/Maus der Methylpyrazol-5-on-Verbindung (subkutan).

Am vierten Tag wurde den vorbehandelten Mäusen (und den Kontrollmäusen) jeweils eine wie vorstehend beschrieben zubereitete Suspension von 5 x 10⁴ B-16ₐ-Tumorzellen über die Schwanzvene injiziert. Die Kontrollmäuse und die behandelten Mäuse wurden unter identischen Bedingungen der Temperatur, Lichteinwirkungsdauer, Ernährung etc. gehalten. Die Mäuse wurden 14 Tage nach erfolgter Schwanzvenen-Injektion getötet, und ihre Lungengewebe wurden untersucht. Die Wirkung des Injizierens der Pyrazolon-Verbindung in die Mäuse eine Stunde vor der Injektion der B-16 a -Tumorzellen wurde ebenfalls untersucht.

Wie aus den in Tabelle 1 zusammengestellten Daten zu ersehen ist, ist die Verabreichung der Pyrazolon-Verbindung in der Weise wirksam, daß dadurch die Zahl der Lungen-Tumorkolonien, d.h. die Metastase, in drastischer Weise vermindert wird, und zwar sowohl auf dem Niveau von 0,08 mg wie auf dem von 0,02 mg.

Es wurde vermutet, daß die vorliegende Pyrazolon-Verbindung die Freisetzung von Prostacyclin stimuliert [siehe The Lancet, Seiten 518-520 (10. März 1979)J. Es wird angenommen, daß die antithrombotische Wirkung des Prostacyclins durch eine Erhöhung des Spiegels der cyclischen Adenosin-3',5'-phosphorsäure (cAMP) in den Plättchen vermittelt wird. Es ist ebenfalls bekannt, daß als Phosphodiesterase-Inhibitoren bekannte Verbindungen die Spaltung von cAMP verlangsamen. Dementsprechend wäre zu erwarten, daß Phosphodiesterase-Inhibitoren, indem sie die Spaltung der cAMP verlangsamen, die antithrombotische Wirkung eines antithrombotischen Mittels, das durch diesen Mechanismus wirkt, potenzieren. Da Plättchen ebenfalls am Mechanismus der Metastase beteiligt sein können, wurde die Wirkung eines wohlbekannten Phosphodiesterase-Inhibitors, des Theophyllins, auf einen potentiellen Synergismus mit der Pyrazolon-Verbindung untersucht.

Obwohl die Ergebnisse darauf hindeuten, daß die antimetastatische Wirkung durch Theophyllin verstärkt worden sein kann, wurde wegen des mit dem Versuch verbundenen Standard-Fehlers ein Synergismus nicht sicher nachgewiesen.

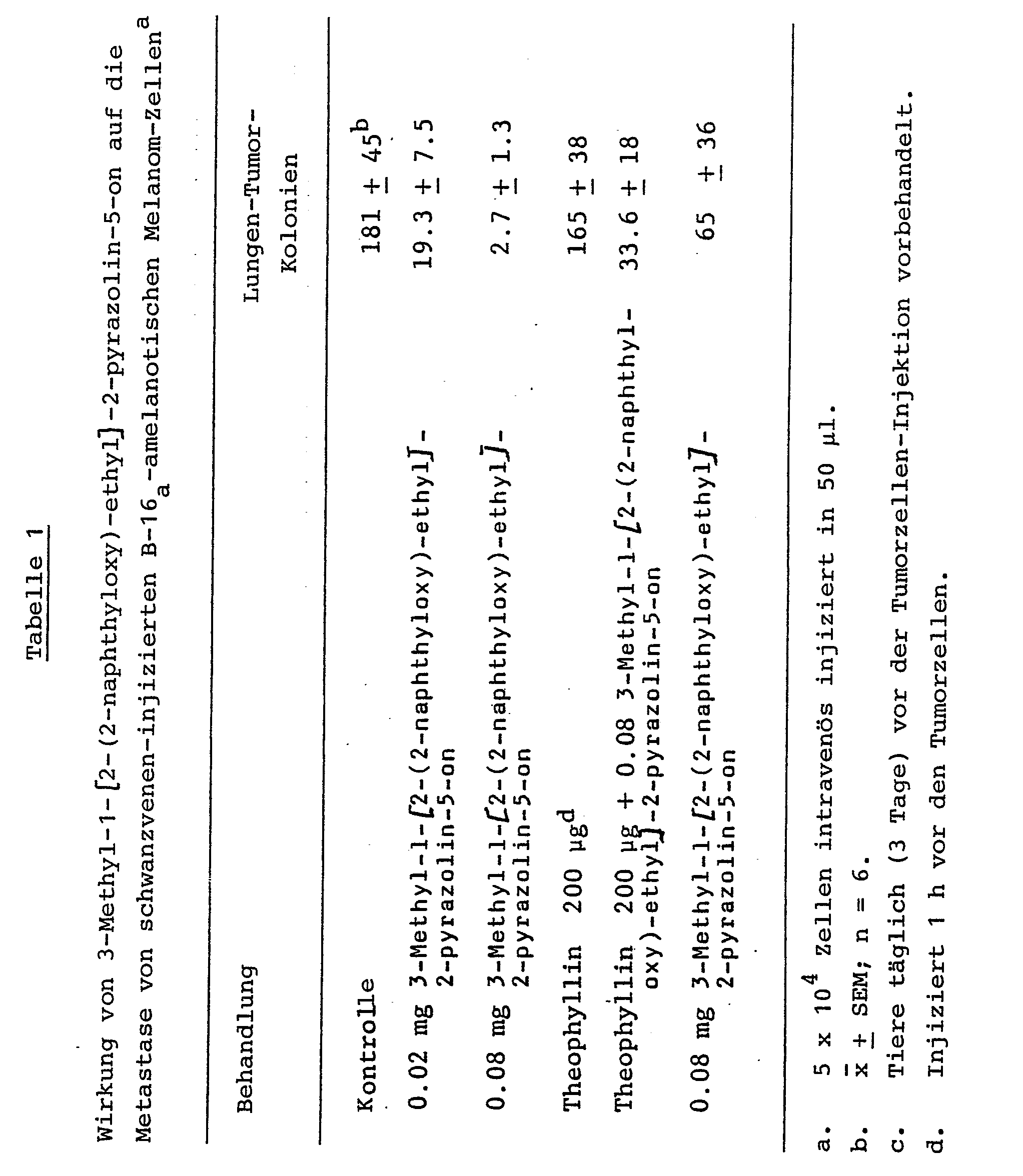

### Beispiel 2

Die Wirkung der Verabreichung der Pyrazolon-Verbindung über einen längeren Zeitraum hinweg auf die Zahl der metastatischen Lungen-Kolonien des B-16 ₐ und des Lewis-Lungen-Karzinoms wurde wie nachstehend beschrieben bestimmt.

3 mg 3-Methyl-1-[2-(2-naphthyloxy)-ethyl]-2-pyrazolin-5-on wurden in 0,6 ml Ethylalkohol gelöst, und die Lösung wurde mit konzentrierter Natronlauge auf pH 9,5 eingestellt.

Syngeneischen C57BL/6J Wirts-Mäusen wurde eine Suspension von 1,8 x 10⁵ B-16 a -Zellen subkutan injiziert, die wie vorstehend beschrieben hergestellt wurde. Einer anderen Reihe syngeneischer C57BL/6J Wirts-Mäuse wurde eine Suspension von 1 x 10⁵ Lewis-Lungen-Karzinom-Zellen, erhalten wie vorstehend beschrieben, subkutan injiziert. Vom Tage nach der Tumorzell-Injektion ab erhielten die Mäuse 28 Tage lang eine subkutane Injektion einer täglichen Einzeldosis von entweder 0,01 oder 0,08 mg der Pyrazolon-Verbindung. Die Kontroll-Mäuse und die behandelten Mäuse wurden unter identischen Bedingungen der Temperatur, Lichteinwirkungsdauer, Ernährung etc. gehalten. Die Mäuse, die die B-16 -Tumorzellen injiziert erhalten hatten wurden 25 Tage nach der Injektion der Tumorzellen getötet; die Mäuse, denen die Lewis-Lungen-Karzinom-Zellen injiziert worden waren, wurden 21 Tage nach der Injektion der Tumorzellen getötet.

Die bei der Untersuchung des Lungengewebes erhaltenen experimentellen Daten sind in der folgenden Tabelle 2 zusammengestellt.

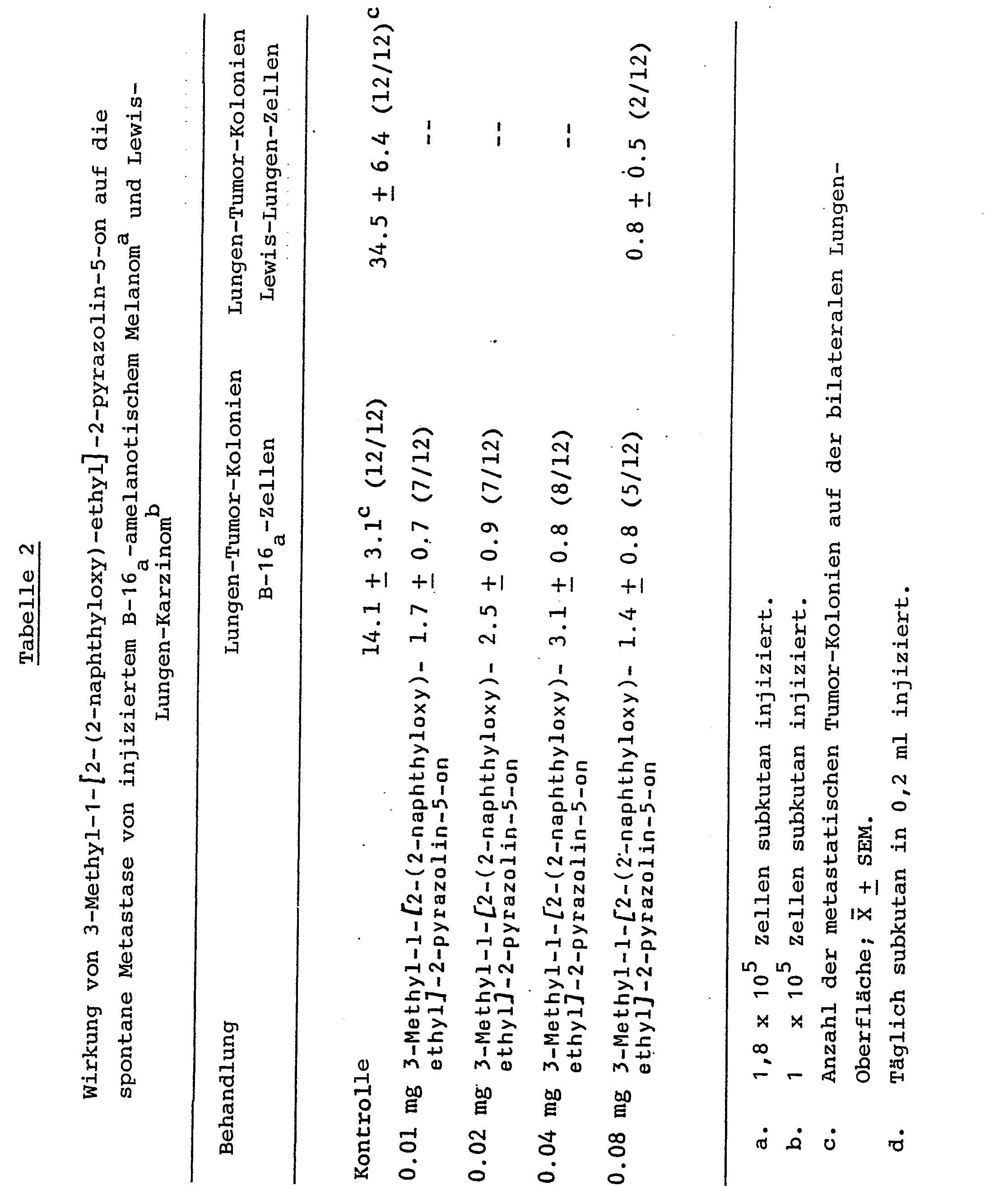

Wie die in Tabelle 2 zusammengefaßten Test-Werte zeigen, wird die Anzahl der metastatischen Lungen-Kolonien sowohl des B-16 -Melanoms als auch des 3LL-Karzinoms durch die Verabreichung der Pyrazolon-Verbindung drastisch vermindert.

Im Hinblick auf die B-16 -Melanom-Metastase schien ein a Dosierungsniveau von 0,01 mg fast genau so wirksam zu sein wie ein Dosierungsniveau von 0,08 mg.

Wie bereits weiter oben dargestellt wird die subkutane Injektion von Tumorzellen und anschließende Entwicklung eines Primär-Tumors, gefolgt von spontaner Metastase, als "volle" Metastase betrachtet. Da die Verfahrensweise des Beispiels 2 diese volle Metastase betraf, war die Zahl der in den Kontrolltieren vorhandenen Lungentumor-Kolonien kleiner als diejenige bei den Kontrolltieren des Beispiels 1, bei dem sich die Metastase aufgrund der Injektion der Tumorzellen in die Schwanzvene entwickelte. Sowohl die Daten in Beispiel 1 als auch die Daten in Beispiel 2 zeigen jedoch, daß die Pyrazolon-Verbindung starke Antimetastasen-Wirksamkeit besitzt.

Die antineoplastische Wirksamkeit der Pyrazolon-Verbindung wurde durch Messung der Thymidin-Aufnahme als Maß für die DNS-Synthese bestimmt.

### Beispiel 3

B-16ₐ- und Lewis-Lungenkarzinom-Zellen wurden wie vor- stehend beschrieben erhalten. Die dispergierten Zellen wurden in sterilen 25 ml-Erlenmeyer-Kolben aus Kunststoff auf eine Konzentration von 1 x 10⁵ Zellen/ml in MEM verdünnt. Tritium-markiertes Thymidin (³H) mit einer spezifischen Aktivität von 1,85 x 10⁻⁸ bis 2,9_{6 x} 10⁻⁸ s⁻¹/mmol (50-80 Ci/mmol) wurde mit 7,4 x 10⁻¹⁶ s⁻¹ /ml (2 µCi/ml) zu jedem ml der Zellsuspension hinzugefügt.

Zu einer Reihe von Kolben, die die B-16 - und Lewis-Lungenkarzinom-Zellen enthielten, wurde die Pyrazolon-Verbindung in Mengen von 0,1 , 1,0 , 10 und 25 µg/ml des Gesamtvolumens hinzugefügt. Die Kolben wurden dann zusammen mit Kontrollkolben der Tumorzellen, die keine Pyrazolon-Verbindung enthielten, bei 37°C in einem Dubnoff-Stoffwechsel-Schüttler (90 Schwingungen/min) inkubiert.

In Zeitabständen von 4 h und 18 h wurden 4 aliquote Anteile zu 1 ml mit einer Pipette entnommen und in konische 1,5 ml-Polypropylenröhrchen gegeben. Die Zellen wurden 8-minütiges Rotieren in einer Brinkman-"Minizentrifuge" (10 000 g) zu Pellets zentrifugiert. Die überstehende Fraktion wurde entfernt und in einen Behälter für radioaktive Abfälle verworfen. Eine Menge von 1,0 ml kalter Trichloressigsäure (6 Gewichts-Volumen-%) wurde jedem Röhrchen zugesetzt, um die anwesenden Proteine auszufällen, einschließlich der DNS und RNS. Die Röhrchen wurden mit einer Kappe verschlossen und herumgewirbelt, um wie oben beschrieben die Zellen durch Zentrifugieren auseinanderzubrechen. Die erhaltenen Pellets enthalten die säureunlösliche Fraktion mit der DNS. Der die säurelöslichen Anteile enthaltende überstand wurde verworfen. Die Innenseite jedes Röhrchens wurde mit einem Wattebausch-Stäbchen abgewischt, um die überschüssige säurelösliche Radioaktivität zu entfernen. Die Pellets wurden durch Zugabe von 50 µl eines Gewebe-Lösungsvermittlers, im Handel erhältlich bei der Amersham Corporation, Del., Arlington Heights, Illinois, unter der Handelsbezeichnung NCS, in jedes Röhrchen aufgelöst. Die Röhrchen wurden mit Kappen verschlossen und bei annähernd 50°C etwa zwei Stunden lang oder bis die Pellets sich auflösten inkubiert.

Die Spitzen der Röhrchen, die die aufgelösten Pellets enthielten, wurden abgeschnitten, und der Inhalt wurde in Szintillationsampullen überführt. 3 ml einer Szintillations-Zählflüssigkeit mit Xylol im Verhältnis 2 : 1 vermischt wurden in jede Szintillationsampulle hineingegeben. Jede Ampulle wurde verschlossen und geschleudert, und die Menge des durch DNS-Synthese aufgenommenen radioaktiv-markierten Thymidins wurde bestimmt. Die Zählwerte pro Minute wurden mit Hilfe eines Searle-PDS-Computers unter Einsatz der Analyse zur Korrektur des Rückgangs (quench-correction analysis) korrigiert und als prozentuale Anteile der Kontrollwerte angegeben.

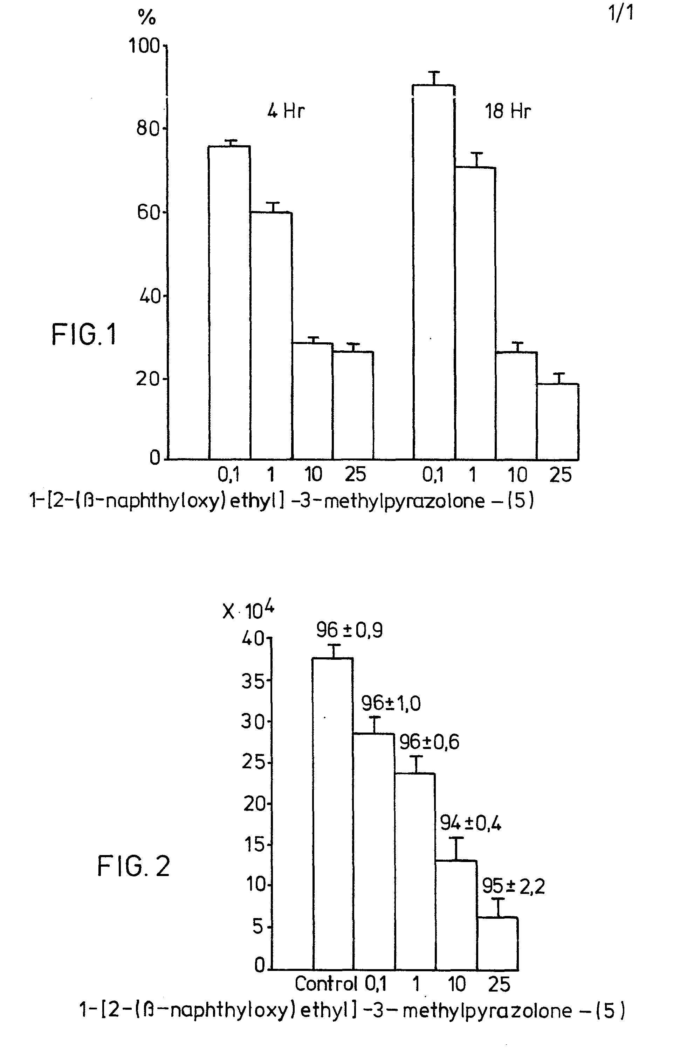

Die erhaltenen Versuchsergebnisse sind in der nachstehenden Fig. 1 dargestellt. Bei jeder Pyrazolon-Konzentration ist die Menge der synthetisierten DNS auf das 100 %-Niveau für die Kontrollprobe bezogen. Wie Fig. 1 zeigt, bewirkte die Pyrazolon-Verbindung eine konzentrationsabhängige Abnahme der DNS-Synthese, wie sie mittels der ³H-Thymidinaufnahme bestimmt wurde. Diese Abnahme der DNS-Synthese zeigt, daß die Pyrazolon-Verbindung antineoplastische Wirksamkeit besitzt.

Der Nachweis für den Einbau des Thymidins in die DNS wurde durchgeführt, wie dies in Biochem. and Biophysical Research Communications, 87, Nr. 3, Seiten 795-801 (1979), beschrieben wurde.

Ein weiterer Beweis für die antineoplastische Wirksamkeit der Pyrazolon-Verbindung wurde durch direkte Messung der Tumorzellen-Proliferation in Gewebekulturen erbracht.

### Beispiel 4

B-16 -Melanom-Zellen, die wie vorstehend beschrieben erhalten worden waren, wurden in 60 mm-Petrischalen mit Gitter (gridded) in einer Dichte von 3 x 10⁴ Zellen pro Platte überimpft. Die Zellen wurden in einem Medium aus MEM, Hank's Basic Salt Solution und 10 % fötalem Kalbsserum, im Handel erhältlich bei Microbiological Associates, Walkersville, Maryland, kultiviert.

Nach 24-stündiger Inkubation bei 37°C wurden die ZellZählungen durch visuelles Zählen in einem Inversionsmikroskop durchgeführt. Das Medium wurde gewechselt und durch das oben bezeichnete.Medium und Pyrazolon in einem von 0,1 bis 25 µg/ml reichenden Konzentrationsbereich ersetzt. Die Kontrollgruppen erhielten nur einen Austausch des Mediums. Danach wurde das Pyrazolon enthaltende Medium jeden zweiten Tag ersetzt. Nach 8 Tagen wurden Zellzählungen wie oben beschrieben durchgeführt, und die Lebensfähigkeit der Zellen wurde nach der weiter oben genannten Vitalfarbstoff-Ausschlußmethode (vital dye exclusive method) bestimmt.

Die erhaltenen Versuchsergebnisse sind in der nachstehenden Fig. 2 dargestellt.

Die Daten sind dargestellt als Mittelwert der Zahl der Zellen + Standardfehler aus Parallelversuchen mit sechs Platten. Die Zahl über jedem Querstrich gibt die Lebensfähigkeit der Zellen, bezogen auf eine 100-prozentige Lebensfähigkeit der Zellen, an. Die Daten zeigen, daß die Pyrazolon-Verbindung die Tumorzellen-Proliferation über den gleichen Dosierungsbereich hemmte, über den die Verbindung auch die DNS-Synthese hemmte.

Der Index der Lebensfähigkeit, der im Bereich zwischen 96 + 0,9 und 94 + 0,4 lag, läßt erkennen, daß die antineoplastische Wirkung der Pyrazolon-Verbindung nicht auf Cytotoxizität der Verbindung beruht.

Zur Messung der antineoplastischen Wirkungen der Pyrazolon-Verbindung wurde ein weiterer Versuch in vivo durchgeführt.

### Beispiel 5

3 mg 3-Methyl-1-[2-(2-naphthyloxy)-ethyl]-2-pyrazolin-5-on wurden in 0,6 ml Ethylalkohol gelöst, und die Lösung wurde mit konzentrierter Natronlauge auf einen pH von 9,5 eingestellt.

Syngeneischen C57BL/6J Wirts-Mäusen wurden 1 x 10⁵ Lewis-Lungenkarzinom-Zellen (0,2 ml) subkutan in die Achselhöhlenregion injiziert. Nach einem Zeitraum von 24 Stunden wurde die Pyrazolon-Verbindung 21 Tage lang täglich verabreicht. Die Wirkung der Verabreichung von Theophyllin zusammen mit der Pyrazolon-Verbindung wurde ebenfalls untersucht. Während des Zeitraums wurden die Mäuse unter identischen Bedingungen der Temperatur, Lichteinwirkungsdauer, Ernährung etc. gehalten. Die Mäuse wurden 22 Tage nach der Injektion der Tumorzellen getötet und mittels einer Gesamt-Nekropsie auf die Anwesenheit subkutaner Tumoren untersucht. Vorhandene Tumoren wurden entfernt und auf einer Analysenwaage gewogen.

Die erhalten Versuchsergebnisse sind in der nachstehenden Tabelle 3 zusammengestellt.

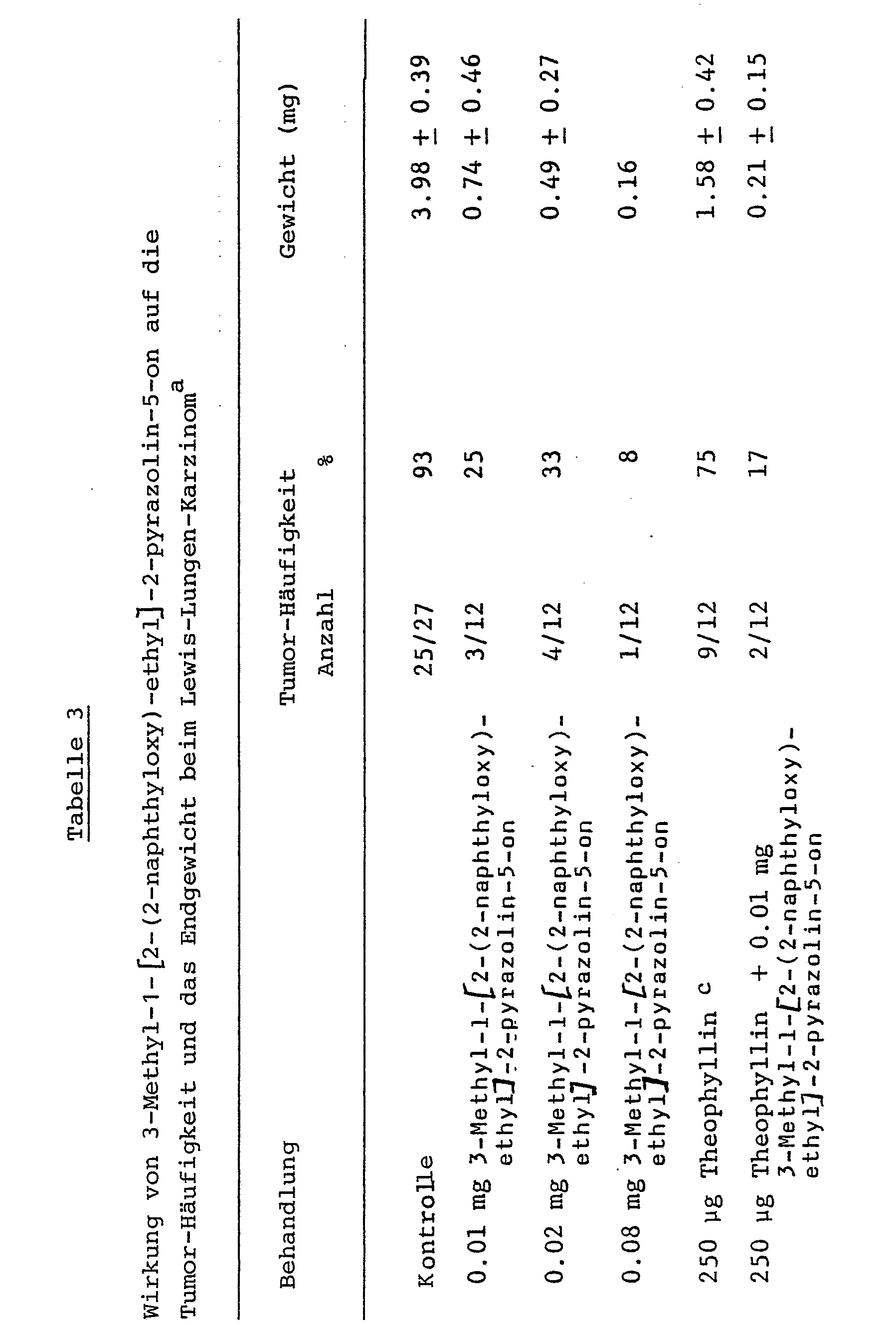

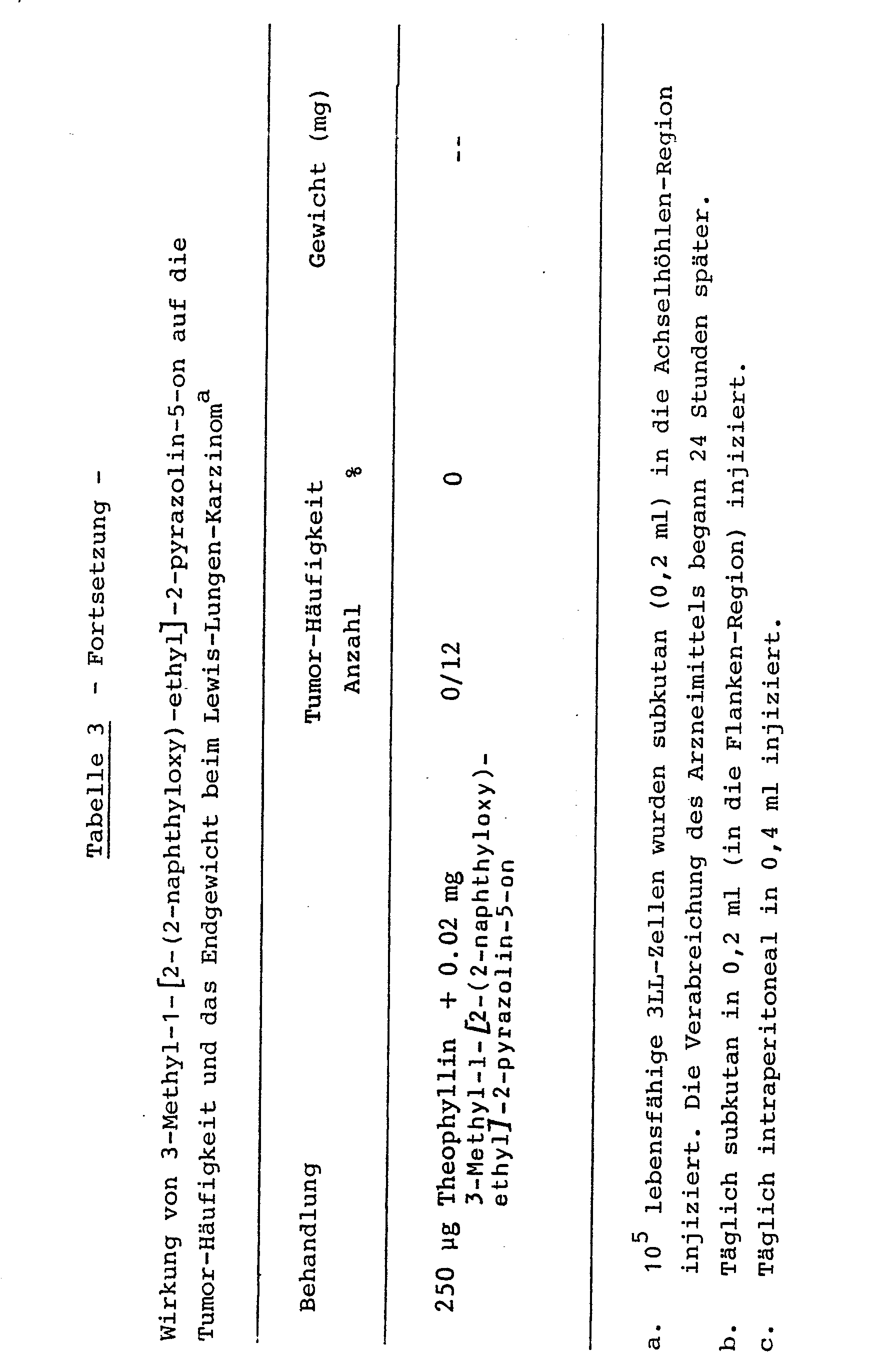

Wie die Versuchsergebnisse in Tabelle 3 zeigen, wurden sowohl die Häufigkeit des Auftretens als auch das Gewicht der Lewis-Lungenkarzinom-Tumoren durch die subkutane Verabreichung der Pyrazolon-Verbindung vermindert; dies zeigt die antineoplastische Wirksamkeit und erhärtet die in vitro-Daten der Beispiele 3 und 4. Theophyllin verstärkte anscheinend die Wirkung der Pyrazolon-Verbindung, möglicherweise durch Erhöhen der Menge des cAMP.

## Patentansprüche

1. Therapeutische Methode zur Reduzierung der Metastase und des neoplastischen Wachstums in Säugern, dadurch gekennzeichnet, daß eine therapeutisch wirksame Menge 3-Methyl-1-[2-(2-naphthyloxy)-ethyl]-2-pyrazolin-5-on oder eines seiner pharmazeutisch unbedenklichen Salze verabreicht wird.

2. Methode nach Anspruch 1, dadurch gekennzeichnet, daß die Verabreichung oral erfolgt.

3. Methode nach Anspruch 1, dadurch gekennzeichnet, daß die Verabreichung parenteral erfolgt.
